# EUROPEAN PATENT SPECIFICATION

(11) **EP 4 705 685 B1**
(45) Date of publication and mention of the grant of the patent: **18.11.2026**
(21) Application number: 24720216.1
(22) Date of filing: 22.04.2024
(51) Int. Cl.: F21V 33/00, A61L 9/20, F21S 8/02, F21V 17/00, F21Y 113/00

(54) **LUMINAIRE PROVIDED WITH A UV LIGHTING MODULE**
LEUCHTE MIT EINEM UVB-BELEUCHTUNGSMODUL
LUMINAIRE POURVU D'UN MODULE D'ÉCLAIRAGE UVB

(30) Priority: 02.05.2023 EP 23170979
(43) Date of publication of application: 11.03.2026
(73) Proprietor: Signify Holding B.V., 5656 AE Eindhoven (NL)
(72) Inventor: VAN BOMMEL, Ties, 5656 AE Eindhoven (NL); BROERSMA, Rémy, Cyrille, 5656 AE Eindhoven (NL)
(74) Representative: Verweij, Petronella Daniëlle
(86) International application number: PCT/EP2024/060894
(87) International publication number: WO 2024/227643

(56) References cited:
- EP-A1- 3 660 385
- KR-A- 20210 124 015
- KR-A- 20230 021 996

## Description

### FIELD OF THE INVENTION

The present invention relates to UV illumination, and particularly to a luminaire having a releasably attached UV lighting module.

### BACKGROUND OF THE INVENTION

UVB light has properties beneficial for humans, such as promoting the production of vitamin B. UVB light is a component of natural sunlight, but in modern society many people are confined to spending much of their time indoors. Artificial UVB light can then be advantageous to maintain a healthy vitamin D production.

Artificial UVB light may be provided in many ways and in many contexts. However, it would be beneficial to provide artificial UVB light as an integrated part of general illumination, e.g. in an office space. In particular, it would be beneficial if existing luminaires could be easily retro-fitted to also provide UVB lighting.

In EP3660385A1 a lighting device is disclosed that includes at least one first light source emitting a visible light, at least one second light source disposed to be spaced apart from the first light source and emitting a light having a sterilization wavelength, and a housing at which the first light source and the second light source are disposed. The first light source is disposed outside an area having an angle corresponding to about 50 percent of a maximum amount of the light emitted from the second light source based on a line substantially perpendicular to a center of the second light source.

### SUMMARY OF THE INVENTION

It is an object of the present invention to provide a UV lighting module which may be retrofitted in existing luminaires.

According to a first aspect of the invention, this and other objects are achieved by a luminaire system comprising a luminaire including a luminaire light source configured to emit white light and a connection slot and a UV lighting module. The UV lighting module emits at least one of UVA, UVB or UVC light and comprises a connection portion configured to be inserted into the connection slot so as to be releasably attachable, a light emitting portion housing a module solid state light source configured to generate module light comprising UV light having a dominant peak wavelength in the 280-320 nm range, and a light exit window configured to exit the module light. A window area of the light exit window is at least 1.5 times as large as an opening area of the connection portion. The UV lighting module has a general shape of a T, wherein the connection portion forms a base of the T, and the UV light emitting portion forms a bar of the T, such that, when the UV lighting module is connected in the connection slot, the light exit window extends on two opposite sides of the connection slot.

White light is here intended broadly but may for example be light having a correlated colour temperature in the range 1500K-6500K, and e.g. a CRI of at least 80.

With this system, a connection slot such as one intended for connection of sensor modules, is used to connect the UV lighting module to a white light luminaire.

Connection slots for connecting various sensors to a luminaire are known e.g. the "upgradeable sensor slot" from Philips. The conventional understanding, and common practice, is that any module connected to such a slot has essentially the same dimensions as the slot. Such existing slots have therefore not been considered for UV-lighting, as a larger exit surface is required to avoid a too high intensity of UV light. The present invention is based on the realization that a UV lighting module can be designed to have a connection portion configured to fit in the connection slot, and a light exit surface which is larger than the slot opening area. By "opening area" is intended a cross section of an opening of the slot in which the connection portion is received.

With this design, a relatively small connection slot (e.g. a few cm wide) may be used to connect a UV lighting module with a significantly larger light exit window). This makes it possible to easily retrofit a luminaire provided with a "sensor slot" with a UV lighting capability.

It is noted that the present invention is equally applicable to module light sources emitting other UV wavelengths than UVB light (i.e. 280-320nm) such as UVA (320-380nm) and/or UVC (100-280nm) light.

Preferably, the area of the light exit window is twice as large as the slot cross section. As an example, the light exit window may have a width in the range 10-60 cm, which is considerably larger than known connection slots.

According to the invention, the UV lighting module has a general shape of a T, wherein the connection portion forms the base of the T, and the UV light emitting portion forms the bar of the T, such that, when the UV lighting module is connected in the slot, the light exit window extends on two opposite sides over the slot. This design is beneficial as it allows the larger size of the light exit window extend only in two directions, thereby making it easier to avoid obstructing light from a luminaire in which the module is mounted.

In some embodiments, the UV light emitting portion further comprises an optical element configured to diffuse or scatter the module light. This may be beneficial, especially if UV radiation is intended to illuminate a particular object, or if it is intended to be homogeneously spread in the ambient.

In some embodiments, the UV light emitting portion comprises a further module solid state light source configured to generate further module light comprising violet light having a (dominant) peak wavelength in the 380-420 nm range.

The module may comprise a controller for individually controlling the luminaire light source and the module light source. The controller may be configured for individually controlling white light emission (e.g. in terms of intensity, color point, correlated color temperature and/or color rendering index) from the luminaire light source and/or module light emission (e.g. intensity) from the module solid state light source. Such a controller may further be functionally connected to a (remotely arranged) indicator (e.g. visual, light, etc.) and be configured to activate the indicator when the module light source is activated.

The module may further comprise a comprise a (proximity) sensor functionally connected to the controller and configured to provide a (proximity) sensor signal e.g. indicating presence of an object (such as a person or animal) in a field of view of the proximity sensor, wherein the controller is configured to control the module solid state light source (and/or the white light emission from the luminaire light source) in dependence on the proximity sensor signal. For example, the controller may be configured to dim, or even deactivate, the module solid state light source when there is no one in the field of view. Alternatively, other sensors may be used such as for example IR-detectors, radar range detectors, sound detectors, etc.

The connection slot is preferably configured to provide a mechanical connection for securing the UV lighting module in the luminaire, and an electrical connection for providing electrical power to the UV lighting module. As an example, the connection slot may have a width in the range 1-8 cm.

In some embodiments, the luminaire includes a central light emitting portion and a peripheral rim at least partly surrounding the central portion, and the connection slot is provided in this rim. This allows mounting a UV lighting module without obstructing the light from the luminaire. Preferably, the light exit window of the UV lighting device is elongated and has a longitudinal axis aligned with said rim. This ensures that the UV light emitting portion including the light exit window does not extend over the light emitting portion of the luminaire.

### BRIEF DESCRIPTION OF THE DRAWINGS

This and other aspects of the present invention will now be described in more detail, with reference to the appended drawings showing embodiments of the present invention.
Fig. 1 is a perspective view of a luminaire provided with a connection slot for receiving a UV module according to an embodiment of the invention.
Fig. 2 shows the UV module in more detail.
Fig. 3 shows a room provided with two luminaires as shown in Fig. 1.

### DETAILED DESCRIPTION

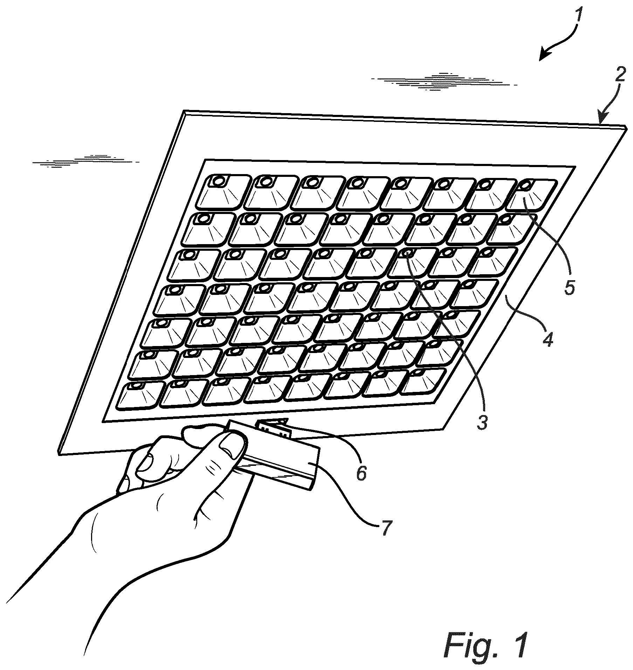

The luminaire 1 in Figure 1 includes a housing 2 and a plurality of light sources 3 arranged in the housing 2. The light sources 3 are typically solid state light sources such as LED light sources.

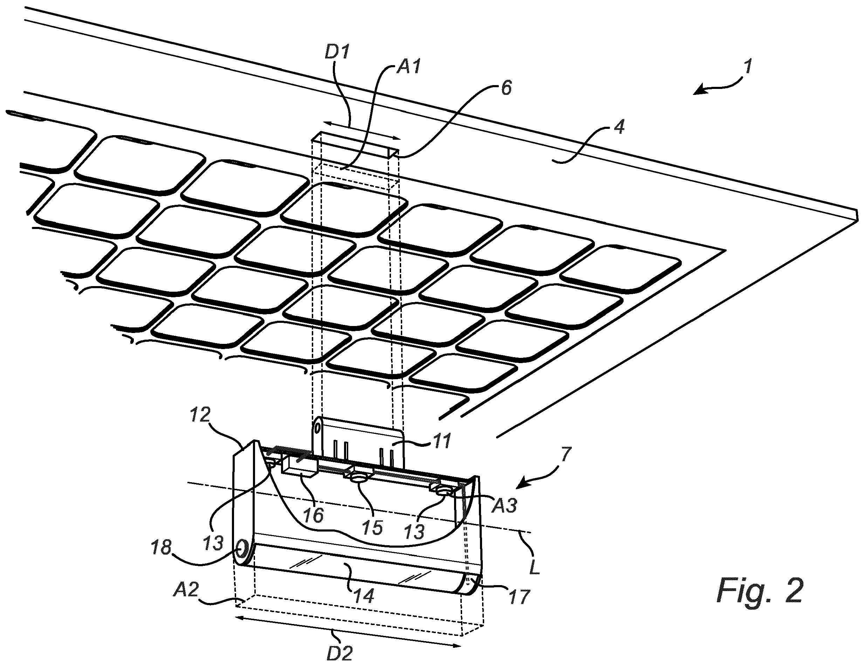

As shown in Figure 1, the housing 2 forms a frame 4 surrounding a light generation portion 5 where the light sources 3 are arranged. The light sources 3 are typically configured to emit white, office-compliant light. In the frame 4 is provided a connection slot 6, configured to receive a plug-in module 7 such that the module 7 is releasably attachable to the luminaire 1. Various types of modules can be connected, including e.g. various sensor modules. Various luminaires with this type of "sensor slot" are sold by Philips ^{®}. Each connection slot 6 has an opening to receive the plug-in module 7, and this opening has a width D1 (i.e. extension in the longest dimension) and an opening area A1 (see Figure 2). As an example, the width may be a few centimeters, e.g. 1-8 cm, preferably 3-5 cm. The area A1 may be in the range 2-20 cm².

Releasable mechanical connection of module 7 in the sensor slot 6 may be provided in a snap-in manner, e.g. by means of a clip or bracket. Electrical connection of the module 7 may be provided by a galvanic plug-and-socket type connection, or by a wireless connection, e.g. through induction. Alternatively, the module 7 may be provided with an on-board power supply, such as a disposable or rechargeable battery (not shown). Details of suitable mechanical and electrical connections are well-known in the art, and will not be discussed in detail herein.

According to aspects of the present invention, the module 7 is a UV lighting module configured to emit UV light. This UV module 7 will be described in more detail with reference to Figure 2.

The UV module 7 has a connection portion 11, configured to be inserted into the connection slot 6 so as to be mechanically secured therein. The UV module 6 further has a UV light emitting portion 12, housing at least one, in the illustrated case two, solid state light source 13 configured to generate UV light (referred to as module light) having a (dominant) peak wavelength in the 280-320 nm range. The light emitting portion 12 has a light exit window 14 configured to exit the UV light into the ambient. The light emitting portion 12 may operate as a light mixing chamber, in which case the exit window 14 is a transparent cover, e.g. plastic or glass. Alternatively, the exit window 14 is simply an opening.

In some embodiments, the light emitting portion 12 further houses at least one solid state light source 15 configured to generate further module light comprising violet light having a (dominant) peak wavelength in the 380-420 nm range.

The window area A2 of the light exit window 14 is at least 1.5 times as large as the opening area A1 of the connection slot 6. As an example, the light exit window can have a width D2 (i.e. extension in the longest dimension) in the range 10-60 cm, preferably 15-25 cm. In other words, several times longer than the connection slot 6. The area A2 may be in the range 10-100 cm². This means that relatively high intensity UV light emitted from the LED(s) 13 may be distributed in the light emitting portion 12, before it is transmitted though the light exit window 14 with less intensity. The LED(s) 13 each have a light output surface area A3 which may be less than 1/5 of the opening area A1. If there are several LEDs 13, then the total light surface area (i.e. the sum of each area A3) may be less than 1/5 of the opening area A1.

According to the invention and as shown in the illustrated embodiment, the UV module 7 has the shape of a T, where the connection portion 11 forms the base of the T and the light emitting portion 12 forms the bar of the T. As shown, when the UV module 7 is inserted into the slot 6, distal portions 12a, 12b of the light emitting portion 12, and consequently the light exit window 14, extend on opposite sides of the slot 6. Further, when the module 7, like in the illustrated case, is aligned with the frame 4, the light exit window 14 will not extend in front of the light emitting portion 5 of the luminaire 1.

The light exit window 14 may be adapted to, or may comprise a separate optical element configured to, diffuse or scatter the light from the LEDs. The light exit window may be curved, to provide an improved distribution of emitted light.

A controller 16 is connected to control the LEDs 13, 15.

In the illustrated example, the LED module 6 further comprises a proximity sensor 17, e.g. an IR sensor or UWB radar sensor. The sensor can be configured to detect when an object is in a field of view of the sensor 17. Alternatively, the sensor can be configured to detect movement within the field of view. The sensor 17 may also be connected to the controller 16, to thereby provide proximity-based control of the emitted light. For example, the controller may be configured to only activate the LEDs 13, 15 in a situation where a living being is determined to be in a field of view of the sensor 17. This may avoid UV emission when there is no-one in a room.

The module 7 may also comprise an indicator 18, e.g. a visible light indicator, connected to the controller 16. The controller can be configured to activate the indicator 18 whenever the UV light source 13 is activated, thereby making a person in the vicinity aware of the UV light emission.

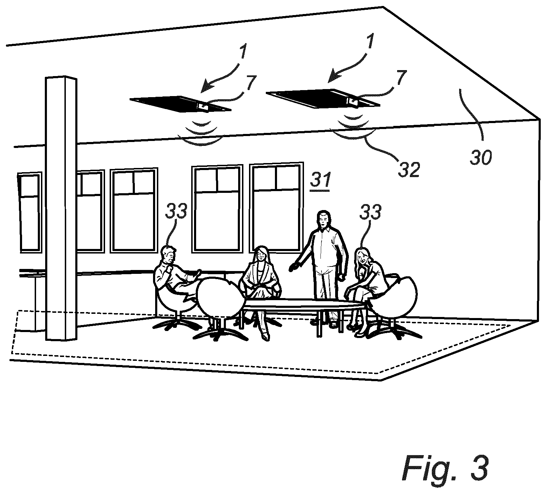

Figure 3 shows two luminaires 1 provided with UV modules 7 suspended in a ceiling 30 of a room 31. Light 32 emitted from the UV modules 7 is incident on two people 33 present in the room 31, thereby ensuring a healthy vitamin D production in these people.

The person skilled in the art realizes that the present invention by no means is limited to the preferred embodiments described above. On the contrary, many modifications and variations are possible within the scope of the appended claims. For example, in an embodiment not claimed, other types of luminaires, provided with appropriate connection slots, can be combined with a UV module used in the invention.

## Claims

1. A luminaire system comprising:
a luminaire (1) including at least one luminaire light source (3) configured to emit white light and a connection slot (6); and
a UV lighting module (7), emitting at least one of UVA, UVB or UVC light, comprising:
- a connection portion (11), configured to be inserted into said connection slot (6) so as to be releasably attachable;
- a light emitting portion (12) housing a module solid state light source (13) configured to generate module light (32) comprising UV light having a dominant peak wavelength in the 100-380 nm range, and a light exit window (14) configured to exit said module light,
wherein a window area (A2) of said light exit window (14) is at least 1.5 times as large as an opening area (A1) of the connection portion (11), **characterised in that**
the UV lighting module (7) has a general shape of a T, wherein the connection portion (11) forms a base of the T, and the UV light emitting portion (12) forms a bar of the T, such that, when the UV lighting module (7) is connected in said connection slot (6), the light exit window (14) extends on two opposite sides of the connection slot (6).

2. The luminaire system according to claim 1, wherein said module solid state light source (13) has a light output surface area (A3) being less than 1/5 of said opening area (A1).

3. The luminaire system according to any one of the preceding claims, wherein the UV light exit window (14) further comprises an optical element configured to diffuse or scatter the module light (32).

4. The luminaire system according to any one of the preceding claims, wherein the light exit window (14) has a width (D2) in the range 10-60 cm.

5. The luminaire system according to any one of the preceding claims, wherein said UV light emitting portion further comprises a further module solid state light source (15) configured to generate further module light comprising violet light having a dominant peak wavelength in the 380-420 nm range.

6. The luminaire system according to any one of the preceding claims, further comprising a controller (16) for individually controlling white light emission from the luminaire light source (3) and module light emission from the module solid state light source.

7. The luminaire system according to claim 6, further comprising an indicator (18) functionally connected to said controller (16), and wherein said controller (16) is configured to activate said indicator when said module solid state light source (13) is activated.

8. The luminaire system according to claim 6 or 7, further comprising a proximity sensor (17) functionally connected to the controller (16) and configured to provide a proximity sensor signal indicating presence of an object in a field of view of the proximity sensor, wherein the controller (16) is configured to control the module solid state light source (13) in dependence on the proximity sensor signal.

9. The luminaire system according to any one of the preceding claims, wherein the connection slot (6) is configured to provide a mechanical connection for releasably securing said UV lighting module in the luminaire (1).

10. The luminaire system according to any one of the preceding claims, wherein the connection slot (6) is configured to provide electrical power to the UV lighting module (7).

11. The luminaire system according to any one of the preceding claims, wherein the luminaire includes a central light generation portion (5) and a peripheral rim (4) at least partly surrounding the central light generation portion (5), and wherein the connection slot (6) is provided in said rim (4).

12. The luminaire system according to claim 11, wherein the light exit window (14) of the UV lighting module (7) is elongated with a longitudinal axis (L) aligned with said rim (4), so that the UV light module (7) does not overlap the central light generation portion (5).

13. The luminaire system according to any one of the preceding claims, wherein the connection slot has a width (D1) in the range 1-8 cm.

## Patentansprüche

1. Leuchtensystem, umfassend:
eine Leuchte (1), einschließend mindestens eine Leuchtenlichtquelle (3), die konfiguriert ist, um weißes Licht abzugeben, und einen Verbindungsschlitz (6); und
ein UV-Beleuchtungsmodul (7), das mindestens eines von UVA-, UVB- oder UVC-Licht emittiert, umfassend:
- einen Verbindungsabschnitt (11), der konfiguriert ist, um in den Verbindungsschlitz eingeführt zu werden (6) , um lösbar befestigbar zu sein;
- einen lichtemittierenden Abschnitt (12), der eine Modulfestkörperlichtquelle (13) aufnimmt, die konfiguriert ist, um Modullicht (32) zu erzeugen, das UV-Licht mit einer dominanten Spitzenwellenlänge im Bereich von 100-380 nm umfasst, und ein Lichtaustrittsfenster (14), das konfiguriert ist, um das Modullicht austreten zu lassen,
wobei eine Fensterfläche (A2) des Lichtaustrittsfensters (14) mindestens 1,5 Mal so groß ist wie eine Öffnungsoberfläche (A1) des Verbindungsabschnitts (11), **dadurch gekennzeichnet, dass**
das UV-Beleuchtungsmodul (7) eine allgemeine Form eines T aufweist, wobei der Verbindungsabschnitt (11) eine Basis des T bildet und der UV-Licht emittierende Abschnitt (12) einen Balken des T bildet, sodass, wenn das UV-Beleuchtungsmodul (7) in dem Verbindungsschlitz (6) verbunden ist, sich das Lichtaustrittsfenster (14) auf zwei gegenüberliegenden Seiten des Verbindungsschlitzes (6) erstreckt.

2. Leuchtensystem nach Anspruch 1, wobei die Modulfestkörperlichtquelle (13) eine Lichtaustrittsoberfläche (A3) aufweist, die kleiner als 1/5 der Öffnungsoberfläche (A1) ist.

3. Leuchtensystem nach einem der vorstehenden Ansprüche, wobei das UV-Lichtaustrittsfenster (14) ferner ein optisches Element umfasst, das konfiguriert ist, um das Modullicht (32) zu diffundieren oder zu streuen.

4. Leuchtensystem nach einem der vorstehenden Ansprüche, wobei das Lichtaustrittsfenster (14) eine Breite (D2) im Bereich von 10-60 cm aufweist.

5. Leuchtensystem nach einem der vorstehenden Ansprüche, wobei der UV-Licht emittierende Abschnitt ferner eine weitere Modulfestkörperlichtquelle (15) umfasst, die konfiguriert ist, um weiteres Modullicht zu erzeugen, das violettes Licht mit einer dominanten Spitzenwellenlänge im Bereich von 380-420 nm umfasst.

6. Leuchtensystem nach einem der vorstehenden Ansprüche, ferner umfassend eine Steuerung (16) zum individuellen Steuern der Weißlichtemission von der Leuchtenlichtquelle (3) und der Modullichtemission von der Modulfestkörperlichtquelle.

7. Leuchtensystem nach Anspruch 6, ferner umfassend eine Anzeige (18), die funktionell mit der Steuerung (16) verbunden ist, und wobei die Steuerung (16) konfiguriert ist, um die Anzeige zu aktivieren, wenn die Modulfestkörperlichtquelle (13) aktiviert ist.

8. Leuchtensystem nach Anspruch 6 oder 7, ferner umfassend einen Näherungssensor (17), der funktional mit der Steuerung (16) verbunden und konfiguriert ist, um ein Näherungssensorsignal bereitzustellen, das die Anwesenheit eines Objekts in einem Sichtfeld des Näherungssensors anzeigt, wobei die Steuerung (16) konfiguriert ist, um die Modulfestkörperlichtquelle (13) in Abhängigkeit von dem Näherungssensorsignal zu steuern.

9. Leuchtensystem nach einem der vorstehenden Ansprüche, wobei der Verbindungsschlitz (6) konfiguriert ist, um eine mechanische Verbindung zum lösbaren Befestigen des UV-Beleuchtungsmoduls in der Leuchte (1) bereitzustellen.

10. Leuchtensystem nach einem der vorstehenden Ansprüche, wobei der Verbindungsschlitz (6) konfiguriert ist, um dem UV-Beleuchtungsmodul (7) elektrische Energie bereitzustellen.

11. Leuchtensystem nach einem der vorstehenden Ansprüche, wobei die Leuchte einen zentralen Lichterzeugungsabschnitt (5) und einen peripheren Rand (4), der den zentralen Lichterzeugungsabschnitt (5) mindestens teilweise umgibt, umfasst, und wobei der Verbindungsschlitz (6) in dem Rand (4) bereitgestellt ist.

12. Leuchtensystem nach Anspruch 11, wobei das Lichtaustrittsfenster (14) des UV-Beleuchtungsmoduls (7) länglich ist, mit einer Längsachse (L), die an dem Rand (4) ausgerichtet ist, sodass das UV-Beleuchtungsmodul (7) den zentralen Lichterzeugungsabschnitt (5) nicht überlappt.

13. Leuchtensystem nach einem der vorstehenden Ansprüche, wobei der Verbindungsschlitz eine Breite (D1) im Bereich von 1-8 cm aufweist.

## Revendications

1. Système de luminaire comprenant :
un luminaire (1) comportant au moins une source lumineuse de luminaire (3) configurée pour émettre une lumière blanche et une fente de connexion (6) ; et
un module d'éclairage UV (7), émettant au moins une lumière UVA, UVB ou UVC, comprenant :
- une partie de connexion (11), conçue pour être insérée dans ladite fente de connexion (6) de façon à pouvoir être fixée de manière amovible ;
- une partie émettrice de lumière (12) abritant une source lumineuse à semi-conducteurs de module (13) configurée pour générer une lumière de module (32) comprenant une lumière UV ayant une longueur d'onde de pic dominante dans la plage de 100 à 380 nm, et une fenêtre de sortie de lumière (14) configurée pour sortir ladite lumière de module,
dans lequel une surface de fenêtre (A2) de ladite fenêtre de sortie de lumière (14) est au moins 1,5 fois plus grande qu'une zone d'ouverture (A1) de la partie de connexion (11), **caractérisé en ce que**
le module d'éclairage UV (7) a une forme générale en T, dans lequel la partie de connexion (11) forme une base du T, et la partie émettrice de lumière UV (12) forme une barre du T, de telle sorte que, lorsque le module d'éclairage UV (7) est connecté dans ladite fente de connexion (6), la fenêtre de sortie de lumière (14) s'étend sur deux côtés opposés de la fente de connexion (6).

2. Système de luminaire selon la revendication 1, dans lequel ladite source lumineuse à semi-conducteurs de module (13) a une superficie de sortie de lumière (A3) inférieure à 1/5 de ladite surface d'ouverture (A1).

3. Système de luminaire selon l'une quelconque des revendications précédentes, dans lequel la fenêtre de sortie de lumière UV (14) comprend en outre un élément optique configuré pour diffuser ou disperser la lumière de module (32).

4. Système de luminaire selon l'une quelconque des revendications précédentes, dans lequel la fenêtre de sortie de lumière (14) a une largeur (D2) dans la plage de 10 à 60 cm.

5. Système de luminaire selon l'une quelconque des revendications précédentes, dans lequel ladite partie émettrice de lumière UV comprend en outre une source lumineuse à semi-conducteurs de module supplémentaire (15) configurée pour générer une lumière de module supplémentaire comprenant de la lumière violette ayant une longueur d'onde de pic dominante dans la plage de 380 à 420 nm.

6. Système de luminaire selon l'une quelconque des revendications précédentes, comprenant en outre un organe de commande (16) pour commander individuellement l'émission de lumière blanche à partir de la source lumineuse de luminaire (3) et l'émission de lumière de module à partir de la source lumineuse à semi-conducteurs de module.

7. Système de luminaire selon la revendication 6, comprenant en outre un indicateur (18) fonctionnellement connecté audit organe de commande (16), et dans lequel ledit organe de commande (16) est configuré pour activer ledit indicateur lorsque ladite source lumineuse à semi-conducteurs de module (13) est activée.

8. Système de luminaire selon la revendication 6 ou 7, comprenant en outre un capteur de proximité (17) fonctionnellement connecté à l'organe de commande (16) et configuré pour fournir un signal de capteur de proximité indiquant la présence d'un objet dans un champ de vision du capteur de proximité, dans lequel l'organe de commande (16) est configuré pour commander la source lumineuse à semi-conducteurs de module (13) en fonction du signal de capteur de proximité.

9. Système de luminaire selon l'une quelconque des revendications précédentes, dans lequel la fente de connexion (6) est conçue pour fournir une connexion mécanique pour fixer de manière amovible ledit module d'éclairage UV dans le luminaire (1).

10. Système de luminaire selon l'une quelconque des revendications précédentes, dans lequel la fente de connexion (6) est configurée pour fournir une alimentation électrique au module d'éclairage UV (7).

11. Système de luminaire selon l'une quelconque des revendications précédentes, dans lequel le luminaire comporte une partie centrale de génération de lumière (5) et un rebord périphérique (4) entourant au moins partiellement la partie centrale de génération de lumière (5), et dans lequel la fente de connexion (6) est prévue dans ledit rebord (4).

12. Système de luminaire selon la revendication 11, dans lequel la fenêtre de sortie de lumière (14) du module d'éclairage UV (7) est allongée avec un axe longitudinal (L) aligné avec ledit rebord (4), de sorte que le module de lumière UV (7) ne chevauche pas la partie centrale de génération de lumière (5).

13. Système de luminaire selon l'une quelconque des revendications précédentes, dans lequel la fente de connexion a une largeur (D1) dans la plage de 1 à 8 cm.
